# EUROPEAN PATENT SPECIFICATION

(11) **EP 3 681 379 B1**
(45) Date of publication and mention of the grant of the patent: **29.11.2023**
(21) Application number: 18782798.5
(22) Date of filing: 13.09.2018
(51) Int. Cl.: A61B 90/00, A61B 5/24, A61B 5/30, A61B 5/316, A61B 5/389, A61B 5/00

(54) **SYSTEM AND METHOD FOR PROVIDING INFORMATION ABOUT THE NEUROMUSCULAR SYSTEM IN AUGMENTED REALITY**
SYSTEM UND VERFAHREN ZUR BEREITSTELLUNG VON INFORMATIONEN ÜBER DAS NEUROMUSKULÄRE SYSTEM IN DER ERWEITERTEN REALITÄT
SYSTÈME ET PROCÉDÉ DESTINÉ À FOURNIR DES INFORMATIONS CONCERNANT LE SYSTÈME NEUROMUSCULAIRE EN RÉALITÉ AUGMENTÉE

(30) Priority: 15.09.2017 IT 201700103440
(43) Date of publication of application: 22.07.2020
(73) Proprietor: Politecnico di Torino, 10129 Torino (IT)
(72) Inventor: GAZZONI, Marco, 10137 Torino (IT); CERONE, Giacinto Luigi, 10134 Torino (IT)
(74) Representative: Metroconsult Srl
(86) International application number: PCT/IB2018/057009
(87) International publication number: WO 2019/053627

(56) References cited:
- MAX ORTIZ-CATALAN ET AL: "Treatment of phantom limb pain (PLP) based on augmented reality and gaming controlled by myoelectric pattern recognition: a case study of a chronic PLP patient", FRONTIERS IN NEUROSCIENCE, vol. 8, 1 January 2014 (2014-01-01), XP055353012, DOI: 10.3389/fnins.2014.00024
- YEE MON AUNG ET AL: "Augmented reality-based RehaBio system for shoulder rehabilitation", INTERNATIONAL JOURNAL OF MECHATRONICS AND AUTOMATION, vol. 4, no. 1, 1 January 2014 (2014-01-01) , pages 52-62, XP055468027, ISSN: 2045-1059, DOI: 10.1504/IJMA.2014.059774
- MA MENG ET AL: "Interactive Mixed Reality for Muscle Structure and Function Learning", 14 August 2016 (2016-08-14), ECCV 2016 CONFERENCE; [LECTURE NOTES IN COMPUTER SCIENCE; LECT.NOTES COMPUTER], SPRINGER INTERNATIONAL PUBLISHING, CHAM, PAGE(S) 117 - 128, XP047394902, ISSN: 0302-9743 ISBN: 978-3-319-69952-3 [retrieved on 2016-08-14] section 2.2 figures 3, 6
- MAX ORTIZ-CATALAN ET AL: "BioPatRec: A modular research platform for the control of artificial limbs based on pattern recognition algorithms", SOURCE CODE FOR BIOLOGY AND MEDICINE, BIOMED CENTRAL LTD, LO, vol. 8, no. 1, 18 April 2013 (2013-04-18), page 11, XP021152620, ISSN: 1751-0473, DOI: 10.1186/1751-0473-8-11

## Description

The present invention relates to a system and a method for providing information about an individual's neuromuscular system in augmented reality.

In particular, the invention relates to an augmented reality system for neuromuscular applications, adapted to acquire and display information about the electric activity of the muscles during a contraction, which may be useful in many situations, ranging from clinical evaluation of muscle activation levels and times to biofeedback provided to the patient during the rehabilitation phase.

Systems are known which provide information about an individual's neuromuscular system, wherein muscle activation is detected by means of electrodes and is typically displayed in different forms (signal over time, bar having a height proportional to the signal amplitude) on a PC screen.

However, these systems are not satisfactory since they have some limitations, such as, for example: during a rehabilitation session, the physiotherapist typically evaluates muscular force and activation by palpation, and the observation of the EMG signal, presented as a graph on a PC screen, does not allow for simultaneous interaction with the patient; for biofeedback applications, the visualization of the EMG signal in different forms on a PC screen does not allow for immediate perception of the activated muscles and of the generated muscular activity, resulting in task learning difficulties and a less effective treatment.

A method for treating phantom limb pain by means of a system based on augmented reality is disclosed in Ortiz-Catalan et al., "Treatment of phantom limb pain(PLP) based on augmented reality and gaming controlled by myoelectric pattern recognition: a case study of achronic PLP patient ", FRONTIERS IN NEUROSCIENCE, vol. 8, 25 February 2014(2014-02-25).

Specifically, the method relies on giving the patient the illusion of a restored limb by showing on a video screen a real time image of the patient's body enhanced by augmented reality (i.e., showing the real-time image of the patient augmented with a virtual limb joined with the termination of the patient's stump). The motion prediction of the missing limb is inferred by collecting myoelectric signals by means of electrodes placed all over the patient's stump; the movements of the missing limb requested by the patient are then determined as a function of the myoelectric signals. Special markers are then used to identify the location of the termination of the stump so as to superimpose the virtual limb in the correct place in the final augmented reality image.

A system for upper-limb rehabilitation is disclosed in Aung et al., "Augmented realitybased RehaBio system for shoulder rehabilitation", INTERNATIONAL JOURNAL OF MECHATRONICS AND AUTOMATION, vol. 4, no. 1, January 2014 (2014-01), pages 52-62. In particular, the system comprises a rehabilitation exercise module based on augmented reality and a biofeedback simulation module for providing the therapist with biofeedback information of patient's muscle performance and activities.

An interactive mixed reality system for learning the structure and function of the muscles associated with an upper extremity is disclosed in MA MENG ET AL: "Interactive Mixed Reality for Muscle Structure and Function Learning", 14 August 2016 (2016-08-14), ECCV 2016 CONFERENCE. In particular, an augmented reality view is generated by superimposing a virtual model of an arm to a real-time video stream.

MAX ORTIZ-CATALAN ET AL: "BioPatRec: A modular research platform for the control of artificial limbs based on pattern recognition algorithms", SOURCE CODE FOR BIOLOGY AND MEDICINE, BIOMED CENTRAL L TO, LO, vol. 8, no. 1, 18 April 2013 (2013-04-18), page 11, discloses an open source software for processing and pattern recognition of myoelectric signals for prosthetic control.

This known data presentation also makes it difficult for a clinician/physiotherapist to integrate tactile, kinematic and electrophysiological information during a treatment.

It is the object of the present invention to solve the above-mentioned problems of the prior art by providing a system and a method for providing information about the neuromuscular system in augmented reality which allows displaying, projected on the skin that covers the muscle, the electric activity generated by a muscle contraction, resulting in better reality perception, easier task learning, and increased treatment effectiveness.

When used by a clinician/physiotherapist, the system for providing information about the neuromuscular system in augmented reality according to the invention also allows for immediate integration of tactile, kinematic, anatomical and electrophysiological information, thus simplifying and optimizing treatments like, for example, the following: 1. rehabilitation exercises: in addition to the muscle contraction information obtained by means of standard techniques (e.g. muscle palpation), the physiotherapist can also use supplementary information about electric muscular activation, without distracting his/her own attention from the patient or having to operate in a different sphere, 2. injection of botulinum toxin: the choice of the injection site can be guided by the identification of the muscle innervation zone, displayed in augmented reality on the skin over the muscle.

These and other objects and advantages of the invention, which will become apparent from the following description, are achieved through a system and a method for providing information about the neuromuscular system in augmented reality as described in the independent claims. Some preferred embodiments and non-obvious variants of the present invention are set out in dependent claims.

The present invention will be described in detail below through a preferred embodiments thereof, which is only provided by way of non-limiting example, with reference to the annexed drawings, wherein:

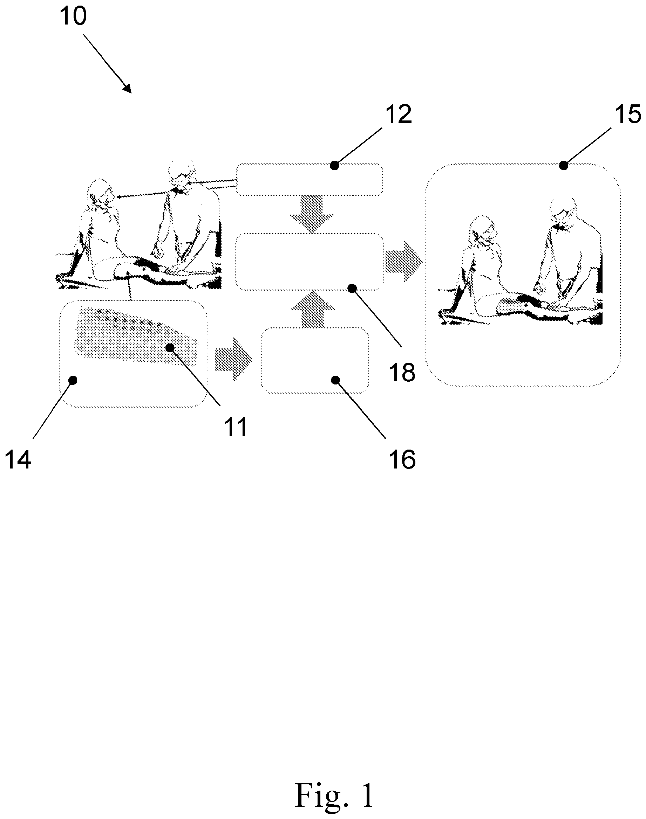

- Figure 1 is a schematic view of a system for providing information about the neuromuscular system in augmented reality according to the present invention; and

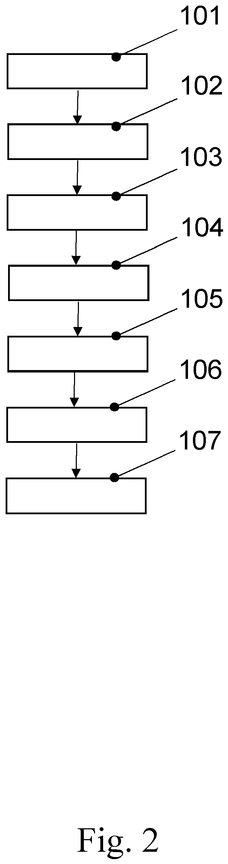

- Figure 2 is a block diagram of a method for providing information about the neuromuscular system in augmented reality according to the present invention.

With reference to the drawings, the system 10 for providing information about the neuromuscular system in augmented reality according to the invention comprises:
- a video shooting system 12 adapted to shoot a video of a region of interest of the patient/individual, or of the whole body thereof, preferably by focusing on the region comprising one or more muscles the electric activity of which has to be detected;
- an electromyographic (EMG) signal acquisition system 14 for acquiring surface electromyographic signals, generated by a muscle contraction, by using one or more sets of electrodes, each one including at least two electrodes 11 placed on the skin that covers said one or more muscles, the electric activity of which has to be detected;

- an electronic conditioning unit 16 adapted to filter, amplify and transmit to a processing and integrating system 18 the surface EMG signal acquired by the electromyographic signal acquisition system 14;
- a data processing and integrating system 18 adapted to:
   1. process every video frame received from the video shooting system 12, identifying the presence of EMG acquisition systems 14 within the frame;
   2. process the epoch of the EMG signal (corresponding to one or more frames of the video signal, depending on the frequency at which one wants to refresh the information provided to the patient/operator/clinician), received from each group of acquisition electrodes 11, and extract information about the neuromuscular system, such as, for example, an amplitude indication (e.g. ARV or RMS), a frequency indication (MNF or MDF), the conduction speed of muscular fibers, or anatomical information which can be extracted by using multi-channel acquisition systems (such as, for example, the signal amplitude distribution on the muscle surface, the position of the innervation zone, the direction of the fibers, the position of the tendons, the length of the muscular fibers, etc.);
   3. associate the groups of EMG acquisition electrodes 11 identified in step 1 with the information about the neuromuscular system processed in step 2;
- an augmented reality system 15 adapted to display on displaying means, superimposed on the acquisition systems 14 identified by the data processing and integrating system 18 in the above-described step 1, the information about the neuromuscular system extracted by the data processing and integrating system 18 in step 2. The information can be represented in different ways: by way of example, by displaying the estimated EMG activity through amplitude or frequency variables by means of a color code (e.g. blue for low EMG activity, red for high EMG activity), or by superimposing on the acquisition system an avatar whose behavior/dimensions/other features are controlled as a function of the value assumed by the variables, or by virtually reconstructing the anatomy of the muscle.

For example, the video shooting system 12 is implemented as a video camera, a webcam, or a video shooting system integrated into smart glasses.

Preferably, the electromyographic signal acquisition system 14 is implemented through the use of one or more pairs of electrodes 11 (bipolar acquisition) placed on the skin that covers one or more muscles, or one or more multi-channel acquisition systems 14 (arrays or matrices of electrodes), and the electrodes may be dry, pre-gelled, fabric-based electrodes or the like.

Preferably, the electronic conditioning unit 16 comprises a conditioning (filtering) module, a surface EMG signal amplification module, and a (wired or wireless) transmission module, and may be either a bipolar or a multi-channel system.

Preferably, the data processing and integrating system 18 comprises memory modules and at least one microprocessor adapted to process every video frame received from the video shooting system, identifying the presence of EMG acquisition systems within the frame, e.g. via marker identification, and adapted to associate each identified marker with the processed information.

For example, the augmented reality system 15 is adapted to display the information about the neuromuscular system, extracted by the data processing and integrating system 18, on displaying means consisting of a screen of a PC or a tablet or a smartphone, or on the lenses of smart glasses; preferably, the augmented reality system 15 is adapted to display the video frame received from the video shooting system 12 and, superimposed on the acquisition systems 14 identified by the data processing and integrating system 18, the information extracted by the data processing and integrating system 18.

The following will describe the operation of the system 10 for providing information about the neuromuscular system in augmented reality according to the invention.

In a first step 101, the video shooting system 12 shoots a video of at least one region of interest of the patient/individual, preferably the region comprising one or more muscles the electric activity of which has to be detected.

In a second step 102, the electromyographic signal acquisition system 14 (bipolar or multi-channel EMG) acquires surface electromyographic signals, generated by a muscle contraction, from one or more muscles by using the group of at least two electrodes 11 applied on the skin that covers said one or more muscles.

In a third step 103, the electronic conditioning unit 16 performs the conditioning (filtering), amplification and transmission of the surface EMG signal.

In a fourth step 104, the data processing and integrating system 18 processes every video frame received from the video shooting system 12, identifying the presence of EMG acquisition systems 14 within the frame.

In a fifth step 105, the data processing and integrating system 18 processes the epoch of the EMG signal received from each group of acquisition electrodes 11, and extracts therefrom information about the neuromuscular system.

In a sixth step 106, the data processing and integrating system 18 associates the acquisition systems 14 identified in the fourth step 104 with the information about the neuromuscular system processed in the fifth step 105.

In a seventh step 107, the augmented reality system 15 displays on displaying means, superimposed on the acquisition systems 14 identified by the data processing and integrating system 18 in step 104, the information about the neuromuscular system extracted by the data processing and integrating system 18 in step 105.

Advantageously, the system 10 for providing information about the neuromuscular system in augmented reality according to the invention allows acquiring surface electromyographic signals from one or more muscles by using a group of at least two electrodes 11, and processing the signals in order to extract information about muscular activation and the peripheral characteristics of the muscle and of its central control, providing such information in visual form superimposed on the image acquired by the video shooting system 12.

Furthermore, the word "comprising" does not exclude the presence of elements and/or steps other than those listed in the claims. The article "a" or "an" before an element does not exclude the presence of a plurality of such elements. The simple fact that some features are mentioned in different dependent claims does not indicate that a combination of such features cannot be used to advantage.

## Claims

1. System (10) for providing information about the neuromuscular system in augmented reality, comprising:
- a video shooting system (12) adapted to shoot a video of a region of interest of an individual, comprising one or more muscles the electric activity of which has to be detected;
- an electromyographic signal acquisition system (14) adapted to acquire surface electromyographic signals, generated by a muscle contraction, by using a group of at least two electrodes (11) placed on the skin that covers said one or more muscles;
- an electronic conditioning unit (16) adapted to filter, amplify and transmit the surface electromyographic signal acquired by the electromyographic signal acquisition system (14);
- a data processing and integrating system (18) adapted to:
• process every video frame received from the video shooting system (12), identifying the presence of electromyographic signal acquisition systems (14) within the frame;
• process the EMG signal received from each group of acquisition electrodes (11) and extract information about the neuromuscular system;
• associate the identified groups of electrodes (11) with the processed information about the neuromuscular system;
- an augmented reality system (15) adapted to display on displaying means, superimposed on the acquisition systems (14) identified by the data processing and integrating system (18), the information about the neuromuscular system extracted by the data processing and integrating system (18).

2. System (10) for providing information about the neuromuscular system in augmented reality according to claim 1, **characterized in that** the information about the neuromuscular system extracted by the data processing and integrating system (18) is an indication of amplitude, frequency, conduction speed of muscular fibers, or anatomical information which can be extracted by using multi-channel acquisition systems.

3. System (10) for providing information about the neuromuscular system in augmented reality according to claim 1 or 2, **characterized in that** the information about the neuromuscular system extracted by the data processing and integrating system (18) can be represented by displaying the estimated EMG activity through amplitude or frequency variables by means of a color code, or by superimposing on the acquisition system an avatar whose characteristics are controlled as a function of the value assumed by the variables, or by virtually reconstructing the anatomy of the muscle.

4. System (10) for providing information about the neuromuscular system in augmented reality according to any one of the preceding claims, **characterized in that** the video shooting system (12) is implemented through a video camera, a webcam, or a video shooting system integrated into smart glasses.

5. System (10) for providing information about the neuromuscular system in augmented reality according to any one of the preceding claims, **characterized in that** the electromyographic signal acquisition system (14) is implemented through the use of one or more pairs of electrodes (11) placed on the skin that covers one or more muscles, or one or more multi-channel acquisition systems (14).

6. System (10) for providing information about the neuromuscular system in augmented reality according to any one of the preceding claims, **characterized in that** the electronic conditioning unit (16) comprises a module for conditioning and a module for amplifying the surface EMG signal, and is a bipolar or multi-channel system.

7. System (10) for providing information about the neuromuscular system in augmented reality according to any one of the preceding claims, **characterized in that** the data processing and integrating system (18) is adapted to process every video frame received from the video shooting system (12), identifying the presence of an electromyographic signal acquisition system (14) within the frame through identification of the acquisition systems, and is adapted to associate each identified acquisition system with the processed information.

8. System (10) for providing information about the neuromuscular system in augmented reality according to any one of the preceding claims, **characterized in that** the augmented reality system (15) is adapted to display the information about the neuromuscular system, extracted by the data processing and integrating system (18), on displaying means consisting of a screen of a PC or a tablet or a smartphone, or on lenses of smart glasses.

9. System (10) for providing information about the neuromuscular system in augmented reality according to any one of the preceding claims, **characterized in that** the augmented reality system (15) is adapted to display the video frame received from the video shooting system (12) and, superimposed on the acquisition systems (14) identified by the data processing and integrating system (18), the information extracted by the data processing and integrating system (18).

10. Method for providing information about the neuromuscular system in augmented reality through the use of a system (10) according to any one of the preceding claims, said method comprising the following steps:
- a first step (101), wherein the video shooting system (12) shoots a video of at least one region of interest of an individual, comprising one or more muscles the electric activity of which has to be detected;
- a second step (14), wherein the electromyographic signal acquisition system (14) acquires surface electromyographic signals, generated by a muscle contraction, from one or more muscles by using a group of at least two electrodes (11) applied on the skin that covers said one or more muscles;
- a third step (103), wherein the electronic conditioning unit (16) performs the conditioning, amplification and transmission of the surface electromyographic signal;
- a fourth step (104), wherein the data processing and integrating system (18) processes every video frame received from the video shooting system (12), identifying the presence of electromyographic signal acquisition systems (14) within the frame;
- a fifth step (105), wherein the data processing and integrating system (18) processes the epoch of the EMG signal received from each group of acquisition electrodes (11) and extracts information about the neuromuscular system;
- a sixth step (106), wherein the data processing and integrating system (18) associates the acquisition systems (14) identified in the fourth step (104) with the information about the neuromuscular system processed in the fifth step (105);
- a seventh step (107), wherein the augmented reality system (15) displays on displaying means, superimposed on the acquisition systems (14) identified by the data processing and integrating system (18) in step (104), the information about the neuromuscular system extracted by the data processing and integrating system (18) in step (105).

## Patentansprüche

1. System (10) zur Bereitstellung von Informationen über das neuromuskuläre System in der erweiterten Realität, umfassend:
- ein Videoaufnahmesystem (12), das eingerichtet ist, ein Video von einem interessierenden Bereich eines Individuums aufzunehmen, der einen oder mehrere Muskeln umfasst, deren elektrische Aktivität erfasst werden soll;
- ein elektromyografisches Signalerfassungssystem (14), das eingerichtet ist, elektromyografische Oberflächensignale zu erfassen, die durch eine Muskelkontraktion erzeugt werden, indem eine Gruppe von mindestens zwei Elektroden (11) verwendet wird, die auf der Haut angebracht sind, die den einen oder die mehreren Muskeln bedeckt;
- eine elektronische Aufbereitungseinheit (16), die eingerichtet ist, das vom elektromyographischen Signalerfassungssystem (14) erfasste elektromyographische Oberflächensignal zu filtern, zu verstärken und zu übertragen;
- ein Datenverarbeitungs- und -integrationssystem (18), das eingerichtet ist:
• jedes von dem Videoaufnahmesystem (12) empfangene Videobild zu verarbeiten, wobei das Vorhandensein von elektromyographischen Signalerfassungssystemen (14) innerhalb des Bildes identifiziert wird;
• das von jeder Gruppe von Erfassungselektroden (11) empfangene EMG-Signal zu verarbeiten und Informationen über das neuromuskuläre System zu extrahieren;
• die identifizierten Gruppen von Elektroden (11) mit den verarbeiteten Informationen über das neuromuskuläre System zu verknüpfen;
- ein System (15) für erweiterte Realität, das eingerichtet ist, die von dem Datenverarbeitungs- und -integrationssystem (18) extrahierten Informationen über das neuromuskuläre System auf Anzeigemitteln anzuzeigen, die den von dem Datenverarbeitungs- und -integrationssystem (18) identifizierten Erfassungssystemen (14) überlagert sind.

2. System (10) zur Bereitstellung von Informationen über das neuromuskuläre System in der erweiterten Realität nach Anspruch 1, **dadurch gekennzeichnet, dass** die Informationen über das neuromuskuläre System, die durch das Datenverarbeitungs- und -integrationssystem (18) extrahiert werden, eine Anzeige der Amplitude, der Frequenz, der Leitungsgeschwindigkeit von Muskelfasern oder anatomische Informationen sind, die durch die Verwendung von Mehrkanalerfassungssystemen extrahiert werden können.

3. System (10) zur Bereitstellung von Informationen über das neuromuskuläre System in der erweiterten Realität nach Anspruch 1 oder 2, **dadurch gekennzeichnet, dass** die Informationen über das neuromuskuläre System, die durch das Datenverarbeitungs- und -integrationssystem (18) extrahiert werden, dargestellt werden können, indem die geschätzte EMG-Aktivität durch Amplituden- oder Frequenzvariablen mittels eines Farbcodes angezeigt wird, oder indem dem Erfassungssystem ein Avatar überlagert wird, dessen Eigenschaften in Abhängigkeit von dem von den Variablen angenommenen Wert gesteuert werden, oder indem die Anatomie des Muskels virtuell rekonstruiert wird.

4. System (10) zur Bereitstellung von Informationen über das neuromuskuläre System in der erweiterten Realität nach einem der vorhergehenden Ansprüche, **dadurch gekennzeichnet, dass** das Videoaufnahmesystem (12) durch eine Videokamera, eine Webcam oder ein in eine intelligente Brille integriertes Videoaufnahmesystem implementiert ist.

5. System (10) zur Bereitstellung von Informationen über das neuromuskuläre System in der erweiterten Realität nach einem der vorhergehenden Ansprüche, **dadurch gekennzeichnet, dass** das elektromyographische Signalerfassungssystem (14) durch die Verwendung eines oder mehrerer Paare von Elektroden (11), die auf der Haut platziert sind, die einen oder mehrere Muskeln bedeckt, oder eines oder mehrerer Mehrkanalerfassungssysteme (14) implementiert ist.

6. System (10) zur Bereitstellung von Informationen über das neuromuskuläre System in der erweiterten Realität nach einem der vorhergehenden Ansprüche, **dadurch gekennzeichnet, dass** die elektronische Aufbereitungseinheit (16) ein Modul zur Aufbereitung und ein Modul zur Verstärkung des Oberflächen-EMG-Signals umfasst und ein bipolares oder mehrkanaliges System ist.

7. System (10) zum Bereitstellen von Informationen über das neuromuskuläre System in der erweiterten Realität nach einem der vorhergehenden Ansprüche, **dadurch gekennzeichnet, dass** das Datenverarbeitungs- und -integrationssystem (18) eingerichtet ist, jedes von dem Videoaufnahmesystem (12) empfangene Videobild zu verarbeiten, wobei es das Vorhandensein eines elektromyographischen Signalerfassungssystems (14) innerhalb des Bildes durch Identifizierung der Erfassungssysteme identifiziert, und eingerichtet ist, jedes identifizierte Erfassungssystem mit den verarbeiteten Informationen zu verknüpfen.

8. System (10) zur Bereitstellung von Informationen über das neuromuskuläre System in erweiterter Realität nach einem der vorhergehenden Ansprüche, **dadurch gekennzeichnet, dass** das System (15) für erweiterte Realität eingerichtet ist, die Informationen über das neuromuskuläre System, die von dem Datenverarbeitungs- und -integrationssystem (18) extrahiert wurden, auf Anzeigemitteln, die aus einem Bildschirm eines PCs oder eines Tablets oder eines Smartphones bestehen, oder auf Linsen einer intelligenten Brille anzuzeigen.

9. System (10) zur Bereitstellung von Informationen über das neuromuskuläre System in erweiterter Realität nach einem der vorhergehenden Ansprüche, **dadurch gekennzeichnet, dass** das System (15) für erweiterte Realität eingerichtet ist, das von dem Videoaufnahmesystem (12) empfangene Videobild und, überlagert von den durch das Datenverarbeitungs- und -integrationssystem (18) identifizierten Erfassungssystemen (14), die durch das Datenverarbeitungs- und -integrationssystem (18) extrahierten Informationen anzuzeigen.

10. Verfahren zur Bereitstellung von Informationen über das neuromuskuläre System in der erweiterten Realität durch die Verwendung eines Systems (10) nach einem der vorhergehenden Ansprüche, wobei das Verfahren die folgenden Schritte umfasst:
- einen ersten Schritt (101), bei dem das Videoaufnahmesystem (12) ein Video von mindestens einem interessierenden Bereich eines Individuums aufnimmt, der einen oder mehrere Muskeln umfasst, deren elektrische Aktivität erfasst werden soll;
- einen zweiten Schritt (14), bei dem das elektromyografische Signalerfassungssystem (14) elektromyografische Oberflächensignale, die durch eine Muskelkontraktion erzeugt werden, von einem oder mehreren Muskeln unter Verwendung einer Gruppe von mindestens zwei Elektroden (11) erfasst, die auf der Haut angebracht sind, die den einen oder die mehreren Muskeln bedeckt;
- einen dritten Schritt (103), bei dem die elektronische Aufbereitungseinheit (16) die Aufbereitung, Verstärkung und Übertragung des elektromyographischen Oberflächensignals durchführt;
- einen vierten Schritt (104), bei dem das Datenverarbeitungs- und -integrationssystem (18) jedes von dem Videoaufnahmesystem (12) empfangene Videobild verarbeitet und das Vorhandensein von elektromyographischen Signalerfassungssystemen (14) innerhalb des Bildes identifiziert;
- einen fünften Schritt (105), bei dem das Datenverarbeitungs- und -integrationssystem (18) die Epoche des EMG-Signals verarbeitet, das von jeder Gruppe von Erfassungselektroden (11) empfangen wird, und Informationen über das neuromuskuläre System extrahiert;
- einen sechsten Schritt (106), bei dem das Datenverarbeitungs- und -integrationssystem (18) die im vierten Schritt (104) identifizierten Erfassungssysteme (14) mit den im fünften Schritt (105) verarbeiteten Informationen über das neuromuskuläre System verknüpft;
- einen siebten Schritt (107), bei dem das System (15) für erweiterte Realität auf Anzeigemitteln die Informationen über das neuromuskuläre System, die von dem Datenverarbeitungs- und -integrationssystem (18) in Schritt (105) extrahiert wurden, anzeigt, die den von dem Datenverarbeitungs- und -integrationssystem (18) in Schritt (104) identifizierten Erfassungssystemen (14) überlagert sind.

## Revendications

1. - Système (10) pour fournir des informations concernant le système neuromusculaire en réalité augmentée, comprenant :
- un système de tournage de vidéo (12) conçu pour tourner une vidéo d'une région d'intérêt d'un individu, comprenant un ou plusieurs muscles dont l'activité électrique doit être détectée ;
- un système d'acquisition de signal électromyographique (14) conçu pour acquérir des signaux électromyographiques de surface, générés par une contraction musculaire, à l'aide d'un groupe d'au moins deux électrodes (11) placées sur la peau qui recouvre ledit ou lesdits muscles ;
- une unité de conditionnement électronique (16) conçue pour filtrer, amplifier et transmettre le signal électromyographique de surface acquis par le système d'acquisition de signal électromyographique (14) ;
- un système de traitement et d'intégration de données (18) conçu pour :
• traiter chaque trame vidéo reçue en provenance du système de tournage de vidéo (12), en identifiant la présence de systèmes d'acquisition de signal électromyographique (14) dans la trame ;
• traiter le signal EMG reçu en provenance de chaque groupe d'électrodes d'acquisition (11) et extraire des informations concernant le système neuromusculaire ;
• associer les groupes d'électrodes identifiés (11) aux informations traitées concernant le système neuromusculaire ;
- un système de réalité augmentée (15) conçu pour afficher, sur des moyens d'affichage, en superposition sur les systèmes d'acquisition (14) identifiés par le système de traitement et d'intégration de données (18), les informations concernant le système neuromusculaire extraites par le système de traitement et d'intégration de données (18).

2. - Système (10) pour fournir des informations concernant le système neuromusculaire en réalité augmentée selon la revendication 1, **caractérisé par le fait que** les informations concernant le système neuromusculaire extraites par le système de traitement et d'intégration de données (18) sont des indications d'amplitude, de fréquence, de vitesse de conduction de fibres musculaires, ou d'informations anatomiques qui peuvent être extraites à l'aide de systèmes d'acquisition multicanaux.

3. - Système (10) pour fournir des informations concernant le système neuromusculaire en réalité augmentée selon la revendication 1 ou 2, **caractérisé par le fait que** les informations concernant le système neuromusculaire extraites par le système de traitement et d'intégration de données (18) peuvent être représentées en affichant l'activité EMG estimée par des variables d'amplitude ou de fréquence au moyen d'un code couleur, ou en superposant sur le système d'acquisition un avatar dont les caractéristiques sont commandées en fonction de la valeur prise par les variables, ou en reconstruisant virtuellement l'anatomie du muscle.

4. - Système (10) pour fournir des informations concernant le système neuromusculaire en réalité augmentée selon l'une quelconque des revendications précédentes, **caractérisé par le fait que** le système de tournage de vidéo (12) est mis en oeuvre par l'intermédiaire d'une caméra vidéo, d'une caméra Web ou d'un système de tournage de vidéo intégré à des lunettes intelligentes.

5. - Système (10) pour fournir des informations concernant le système neuromusculaire en réalité augmentée selon l'une quelconque des revendications précédentes, **caractérisé par le fait que** le système d'acquisition de signal électromyographique (14) est mis en oeuvre en utilisant une ou plusieurs paires d'électrodes (11) placées sur la peau qui recouvre un ou plusieurs muscles, ou un ou plusieurs systèmes d'acquisition multicanaux (14).

6. - Système (10) pour fournir des informations concernant le système neuromusculaire en réalité augmentée selon l'une quelconque des revendications précédentes, **caractérisé par le fait que** l'unité de conditionnement électronique (16) comprend un module de conditionnement et un module d'amplification du signal EMG de surface, et est un système bipolaire ou multicanal.

7. - Système (10) pour fournir des informations concernant le système neuromusculaire en réalité augmentée selon l'une quelconque des revendications précédentes, **caractérisé par le fait que** le système de traitement et d'intégration de données (18) est conçu pour traiter chaque trame vidéo reçue en provenance du système de tournage de vidéo (12), en identifiant la présence d'un système d'acquisition de signal électromyographique (14) dans la trame par identification des systèmes d'acquisition, et est conçu pour associer chaque système d'acquisition identifié aux informations traitées.

8. - Système (10) pour fournir des informations concernant le système neuromusculaire en réalité augmentée selon l'une quelconque des revendications précédentes, **caractérisé par le fait que** le système de réalité augmentée (15) est conçu pour afficher les informations concernant le système neuromusculaire, extraites par le système de traitement et d'intégration de données (18), sur des moyens d'affichage consistant en un écran d'ordinateur personnel ou d'une tablette ou d'un téléphone intelligent, ou sur des verres de lunettes intelligentes.

9. - Système (10) pour fournir des informations concernant le système neuromusculaire en réalité augmentée selon l'une quelconque des revendications précédentes, **caractérisé par le fait que** le système de réalité augmentée (15) est conçu pour afficher la trame vidéo reçue en provenance du système de tournage de vidéo (12) et, en superposition sur les systèmes d'acquisition (14) identifiés par le système de traitement et d'intégration de données (18), les informations extraites par le système de traitement et d'intégration de données (18).

10. - Procédé pour fournir des informations concernant le système neuromusculaire en réalité augmentée par utilisation d'un système (10) selon l'une quelconque des revendications précédentes, ledit procédé comprenant les étapes suivantes :
- une première étape (101), dans laquelle le système de tournage de vidéo (12) tourne une vidéo d'au moins une région d'intérêt d'un individu, comprenant un ou plusieurs muscles dont l'activité électrique doit être détectée ;
- une deuxième étape (14), dans laquelle le système d'acquisition de signal électromyographique (14) acquiert des signaux électromyographiques de surface, générés par une contraction musculaire, à partir d'un ou plusieurs muscles en utilisant un groupe d'au moins deux électrodes (11) appliquées sur la peau qui recouvre ledit ou lesdits muscles ;
- une troisième étape (103), dans laquelle l'unité de conditionnement électronique (16) assure le conditionnement, l'amplification et la transmission du signal électromyographique de surface ;
- une quatrième étape (104), dans laquelle le système de traitement et d'intégration de données (18) traite chaque trame vidéo reçue en provenance du système de tournage de vidéo (12), en identifiant la présence de systèmes d'acquisition de signal électromyographique (14) dans la trame ;
- une cinquième étape (105), dans laquelle le système de traitement et d'intégration de données (18) traite l'époque du signal EMG reçu en provenance de chaque groupe d'électrodes d'acquisition (11) et extrait des informations concernant le système neuromusculaire ;
- une sixième étape (106), dans laquelle le système de traitement et d'intégration de données (18) associe les systèmes d'acquisition (14) identifiés dans la quatrième étape (104) aux informations concernant le système neuromusculaire traitées dans la cinquième étape (105) ;
- une septième étape (107), dans laquelle le système de réalité augmentée (15) affiche, sur des moyens d'affichage, en superposition sur les systèmes d'acquisition (14) identifiés par le système de traitement et d'intégration de données (18) à l'étape (104), les informations concernant le système neuromusculaire extraites par le système de traitement et d'intégration de données (18) à l'étape (105).
